# EUROPEAN PATENT SPECIFICATION

(11) **EP 2 243 478 B1**
(45) Date of publication and mention of the grant of the patent: **08.04.2015**
(21) Application number: 08833593.0
(22) Date of filing: 10.09.2008
(51) Int. Cl.: A61K 45/06, A61K 31/222, A61K 31/737

(54) **PHARMACEUTICAL COMPOSITION COMBINING AN ANTIARTHRITIC AGENT AND AN INTERLEUKIN-1 INHIBITING AGENT, WHICH CAN BE USED TO CONTROL AND TREAT OSTEOARTHRITIS AND RELATED DISEASES**
PHARMAZEUTISCHE ZUSAMMENSETZUNG AUS EINER KOMBINATION EINES ANTIARTHRITIS-MITTELS UND EINES INTERLEUKIN-1-HEMMENDEN MITTELS ZUR KONTROLLE UND BEHANDLUNG VON OSTEOARTHROSE UND VERWANDTEN ERKRANKUNGEN
COMPOSITION PHARMACEUTIQUE COMPRENANT LA COMBINAISON D'UN AGENT ANTI-ARTHRITIQUE ET D'UN AGENT INHIBITEUR DE L'INTERLEUKINE 1, UTILE POUR LE CONTRÔLE ET LE TRAITEMENT DE L'OSTÉOARTHROSE ET DE MALADIES ASSOCIÉES

(30) Priority: 26.09.2007 MX 2007011931
(43) Date of publication of application: 27.10.2010
(73) Proprietor: PPTM INTERNATIONAL S.à r.l., 1253 Luxembourg (LU)
(72) Inventor: GARCÍA ARMENTA, María Elena, C.P. 45020 Guadalajara Jalisco (MX); SANTOS MURILLO, Josefina, C.P. 44600 Zapopan Jalisco (MX); ÁLVAREZ OCHOA, Victor Guillermo, C.P. 45222 Zapopan Jalisco (MX)
(74) Representative: Carvajal y Urquijo, Isabel
(86) International application number: PCT/MX2008/000124
(87) International publication number: WO 2009/041799

(56) References cited:
- EP-A1- 1 655 026
- WO-A1-99/40926
- MONGIL E ET AL: "Symptomatic slow acting drugs for osteoarthritis (Sysadoa)", REVISTA DE LA SOCIEDAD ESPANOLA DEL DOLOR 200610 ES, vol. 13, no. 7, October 2006 (2006-10), pages 484-495, XP9142117, ISSN: 1134-8046
- MULERO MENDOZA J.: 'Tratamiento farmacologico of the artrosis.' EXPECTATIVAS AND REALIDADES. REV. CLIN. ESP. vol. 205, no. 4, 2005, pages 168 - 171, XP008114911
- BOURGEOIS, P.: 'Efficacy and tolerability of chondroitin sulfate 1200 mg/day vs chondroitin sulfate 3x400mg/day vs placebo.' OSTEOARTHRITIS AND CARTILAGE. vol. 6, no. SUPP.A, 1998, pages 25 - 30, XP022221990

## Description

### FIELD OF INVENTION

The present invention has application in pharmaceutical industry and discloses a pharmaceutical composition comprising a synergistic combination of an antiarthritic agent: Chondroitin sulphate and an interleukin-1 inhibiting agent: Diacerein, in addition to pharmaceutically acceptable excipients; which are formulated in a single dosage unit to be orally administered, for use in the control and treatment of osteoarthrosis and related diseases

The combination of above mentioned active principles produces a higher synergistic effect when administered in combination with a single dosage unit unlike when they are independently administered, causing benefits such as: lower active principle concentrations comprised in the formulation, smaller administered doses, faster pharmacological action, therapeutic effect enhancement and lower risks that side effects are present.

### BACKGROUND OF INVENTION

Osteoarthrosis (OA) or arthrosis is a heterogeneous pathology group with similar clinical manifestations and common pathological and radiologic changes. Arthrosis is a result of mechanical and biological factors which destabilize the normal coupling between degradation and synthesis, by chondrocytes, of the extracellular matrix of joint cartilage and subchondral bone. Arthrosis may start by a number of factors those which include: genetic, environmental, metabolic and traumatic factors.

It is estimated that 1/3 of individuals older than 35 years show any arthrosis sign, with a prevalence which is increased with age. Hand and knee arthrosis is more common in women, while hip arthrosis prevalence is similar in both sexes. Women between 70 and 89 years old develop symptomatic knee arthrosis in about 1% per year.

Depending on the affected joint, risk factors are different and they may be differentiated among a generalized susceptibility such as: age, osteoporosis, inheritance and sex, and local joint factors such as: traumatisms, anatomical joint alterations and patient's labor occupation.

There are a number of studies which defend the relationship between obesity and knee arthrosis; however, the mechanisms whereby obesity may lead to arthrosis appearance are still unknown.

There are at least three theories: the most acceptable hypothesis is that overweight increases the pressure exerted over a joint and this may induce cartilage breakage, but this theory would not explain a possible relationship between obesity and hand OA.

Similarly, it has been demonstrated that association of knee arthrosis with work demands prolonged and repeated flexions in this joint.

It has been observed that patients with higher bone density have a higher risk to suffer arthrosis. Thus, it has been established that a reduced bone mass may increase absorption bone capacity from juxta-articular bone vibrations thus protecting the joint cartilage.

Its incidence is not only higher in women, but is also more intense and affects to more joints. Topographic pattern shows differences since it is more frequent the metacarpal phalangeal joints and hip in men and distal interphalangeal joints and knees in women.

A remarkable increase in severe arthrosis frequency has been found in elderly people. Correlation is non-linear and increase is exponential from 50 years old. The mechanism causing the association between aging and arthrosis is little known. Within the possible factors which include small anatomical changes in joints and biomechanical and biochemical alterations in articular cartilage compromise cartilage mechanical properties.

There are proofs that chondrocytes cause several oxygen radicals and that oxidative impairment may be relevant. Thus it is established that antioxidant molecules, such as C, E and D vitamins are of benefit for arthrosis.

Arthrosis is a result of a loss of articular function because of a fracture of articular cartilage. Though articular cartilage degradation is the main event in arthrosis pathogenesis, other tissues such as synovial or subchondral bone, participate in the commencement and development of this pathology. Final result is matrix accelerated destruction by enzymes proceeding from chondrocytes and synovial cells, followed by alterations in cartilage repair systems.

The following factors participate in OA or arthritis pathogenesis:
- Mechanical factors: joints which support load are subject to repeated and located pressures. Although one of the periarticular tissue and subchondral bone functions is to dissipate energy in load joints, some mechanical forces are transmitted to the cartilage.
- Enzymatic mechanisms: enzyme groups which develop an essential function in OA are metalloproteases and serinoproteases.
- Synovial tissue: it has been demonstrated synovial tissue inflammation in arthrosis and its participation in articular cartilage destruction and in this articular pathology becoming chronic.
- Clinical manifestations: pain is the most frequent symptom and is located in affected joint. Firstly, pain is unchained when joint is used and improves with rest. As disease progresses, pain is more continuous appearing in rest and even by night interfering with sleep. A correlation between pain intensity and joint structural damage degree is not always available; narrowest correlation is in hip arthrosis followed by that from knee, being worse in hand and in spine cord spinal apophysis.

Pain origin in patients with arthrosis is multifactorial, depending both on joint structures and periarticular structures. Pain causes include: an increase in intra-bone pressure secondary to an intravenous obstruction; periostal stretching secondary to osteophyte formation, - subchondral microfractures; synovial hypertrophy which causes inflammation; capsular distress, ligament distresses and muscle contractures.

Rigidity is another of the symptoms which feature arthrosis, appearing after an inactivity period and morning rigidity may be also present. Rigidity duration is always short in time unlike rigidity in inflammatory diseases. In knee arthrosis for instance, it is smaller than 30 minutes.

Bone crepitation under joint active and passive movement is a characteristic sign; it is appreciated in all the joint movement range. Pain may exist under pressure along the whole articular and periarticular line. It is frequently found a decrease in joint movement range.

A temperature increase is present occasionally in affected joint with several degrees of joint effusion. Deformation is present in cases where OA is very advanced, although a joint instability is frequently found. Periarticular muscle atrophy may be present in advanced stages due to a lack of use or to a reflex inhibition of muscle contraction.

Arthrosis intensity diagnosis and gradation is commonly performed based on clinical data and radiologic image.

The first step for- diagnosis is to determine whether clinical condition shown by patient is due to that joint arthrosis or to other causes.

Next step is to differentiate between patients showing primary arthrosis (idiopathic) and those with secondary arthrosis to other diseases.

Third step is to confirm that patients meet *clinical criteria* for arthrosis, such as those proposed by the American College of Rheumatology. These criteria are useful for orienting in doubtful cases and for homogeneizing patient populations with similar clinical conditions and used mainly in epidemiologic studies. In case of knee arthrosis, classification is based on pain presence in knee according to clinical criteria, crepitations with active movement and morning rigidity of less than 30 minutes of duration. All patients shall refer pain during most of days of prior month to medical visit.

*Radiological criteria* for knee arthrosis classification are based on the presence of radiological osteophytes. Anteroposterior and side radiography of both lead knees shall be performed in the radiological study. Classical radiological signs are: decrease in joint space, osteophytes, subchondral sclerosis, cysts, bone line abnormalities and joint luxations.

The most used grading system for radiological changes was developed by Kellegren and Lawrence in 1957. Ecography and magnetic resonance are image techniques which allow assessing the presence of joint effusion, cartilage thickness and the presence of periarticular pathology.

From a clinical point of view an ecography or magnetic resonance is not justified to be performed in arthrosis diagnosis, but these techniques may be of help to discard other associated pathologies.

Classic laboratory tests for the study of rheumatic diseases are normal in patients with arthrosis. A mildly high globular sedimentation speed may be occasionally present and rheumatoid factor may be positive at low titer; however, both assumptions do not exclude an arthrosis diagnosis in elderly people.

Twenty percent of healthy elder people may have a positive rheumatoid factor slightly increased with age at low titer and globular sedimentation speed. However, any serum or synovial liquid marker is not currently available in patients with arthrosis allowing a diagnosis or follow-up thereof. Significant advancements in the study of arthrosis biological markers which register a biological, physiological or pathological process have been obtained.

In the case of arthrosis, it is important that biological markers are cartilage-specific, or at least as specific as possible (type II collagen and proteoglicans such as aggrecan). These markers may be determined in serum, urine or synovial liquid.

Synovial liquid shows non-inflammatory features being viscous, non-turbid and the cell number is below 2,000/mm. It shall be always observed with a polarized light microscope ruling out the presence of crystals, mainly of dihydrated calcium pyrophosphate.

Complications which may suffer an arthrosic joint may be of different origin:
a) *Inflammatory*: the most frequent is arthritis by microcrystals which occasionally is originated by the association with chronic chondrocalcinosis or pseudogout.
b) *Infectious*: by gram-positive bacteria, mainly by *Staphylococcus aureus.*
   Diagnosis of these two complications shall be suspected when a continuous and significant joint pain is present accompanied by inflammatory joint data, such as synovial effusion and local heat.
c) *Traumatologic*: the presence of degenerative meniscopathy and osteochondritis.

Knee arthrosis is equally present in men and women and frequently of unique location. Main symptom is mechanical pain located at groin and irradiated to thigh front side up to knee. A rear pain located in buttocks or thigh inner or rear side is occasionally present.

Pain may be intense creating a great functional limitation. Rigidity is usual after rest periods. Upon exploration there is a significant decrease in passive motility, especially in flexion and abduction. Atrophy in quadriceps and buttocks may be observed. In advanced stages, the pelvis oscillates towards the healthy side when patient is supported on diseased side and a compensating hyperlordosis may be observed. Evolution may be variable, stable cases may be present for many years while others will promptly require surgery.

Knee arthrosis uses to be more frequent and is associated with obesity. The main symptom is mechanical pain, with rigidity, crepitation and functional impotence which is more severe as disease is more advanced. Depending on the injury location, pain will be global on the sides of rear zone when centered in femoral-tibial compartment and pain will be produced when going up or down stairs and when kneeling down.

Sometimes blockages may exist because of the presence of any intraarticular free body. There are pain and flexion limitation, kneecap displacement and joint tumefaction under exploration. It is useful to prove the presence of angle alterations such as: varus (when knees tend to separate) or valgus (when knees tend to be together).

A constant sign in advanced stages is quadriceps atrophy and a flexion attitude more or less important.

Most of the success in arthrosis treatment lies in a proper diagnosis, that is, in properly ruling out other possible pain causes or pathologies associated with arthrosis.

Treatment must be individual and adjusted to the affected joint. Objectives of arthrosis treatment are: controlling symptomatology, maintaining joint function and a maximum reduction of arthrosis progression. According to these objectives, arthrosis treatment may be classified in two large therapeutic groups:
- Symptom modifying treatments, which are those therapeutical options (whether or not pharmacological) which reduce patient symptomatology (pain).
- Structure modifying treatments, which are those therapeutic options capable of reducing, arresting or reverting joint cartilage destruction.

Pain origin in arthrosis is not fully known. Inflammation may be present and may cause pain because of a direct stimulation of primary afferent nociceptive fibers or by sensitizing these nerve fibers through mechanical or chemical stimuli. Moreover, pain has a central component and situations such as anxiety, depression and social isolation may have influence in perception thereof.

Nowadays we know that arthrosis ethiopathogeny and clinical manifestation are not the same in all joints. Spine cord arthrosis is different from arthrosis affecting limb joints.

Recent advancements in arthrosis ethiopathogeny have achieved that treatment progresses and suffers significant changes. We currently have a therapeutic arsenal below described.
1.- *Non*-*pharmacological treatment.* There are a number of rules that a patient with arthrosis must know. Firstly, disease nature should be explained to lead to positive attitudes. Occupational therapy may perform a significant role in patient education. It is useful to provide single advices about joint economy, teaching measures directed to reduce the load supported by joints. It has been demonstrated that weight loss in obese patients reduces symptomatology and delays the progressive destruction of joint cartilage. The use of a cane helps to mitigate pain, also reducing the risk of falls. Aerobic exercise performs a significant role in joint protection by increasing muscle force and improving blood flow in joint, cartilage nutrition and the risk of joint motility.

Because of that, strengthening the musculature which is next to the joint (quadriceps, knee or hip abductor or extensor muscles) with isometric exercises is also useful.

The use of suitable footwear may help to mitigate pain in lower limb joints. Alterations in alignment (genu varum or valgum) may be also occasionally corrected by introducing into footwear some single side wedges. Heat and cold (diathermy, ultrasound, infrared, paraffin baths, electric pillows) may be useful to release pain caused by arthrosis.
2.- *Pharmacological treatment.* OA pharmacological treatment is divided into two groups: a) symptom modifying drugs targeted to pain control and b) structure modifying drugs targeted to preserve joint cartilage and to slow down disease progress.

Most of the drugs which are currently found in marketplace for treatment of OA comprise an active principle which is independently formulated, which comply with a specific therapeutic activity; however, these drugs, especially the structure modificators which are targeted to preserve the joint cartilage and to slow down disease progress, have a slow activity leading that patient cancels the treatment having complications such as: an increase in symptomatology and an increase in joint structure impairment.

WO 99/40926 discloses different combinations of agents for treating, repairing and preventing damage to connective tissues, in particular for joint diseases such as rheumatoid arthritis or osteoarthritis. The table on page 21 discloses the combination of chondroitin and diacerein. The doses mentioned therein for humans are 75-4000 mg for chondroitin sulfate and 20-3000 mg for diacerein (pages 23 and 24).

MONGIL ET AL: "Symptomatic slow acting drugs for osteoarthritis (Sysadoa)", REVISTA DE LA SOCIEDAD ESPANOLA DEL DOLOR 200610 ES, vol. 13, no. 7, October 2006 (2006-10), pages 484-495 is a review on slow action symptom modifying drugs for osteoarthritis. Both chondroitin sulphate and diacerein are mentioned therein. The dosage mentioned for chondroitin sulphate is 800 mg/day given in a single dose or in two doses of 400 mg each. The dosage of diacerein is 50 - 100 mg/day given in one or two doses of 50 mg each.

### SUMMARY OF INVENTION

In order to provide a pharmaceutical alternative which reduces symptomatology in patients who suffer from OA, such as inflammation and pain, further manifesting a modifying effect in joint structure, slowing down disease progress and reducing the risk of manifesting secondary effects, ) development of present invention was carried out whereby a pharmaceutical composition is disclosed comprising a combination of an antiarthritic agent, namely chondroitin sulphates and an interleukin-1 selective inhibiting agent, namely Diacerein, wherein chondroitin sulphate is present on the formulation on a concentration range from 100.0 mg to 1.2 g and Diacerein from 25.0 mg to 100.0 mg, which act synergistically and formulated in a single dosage unit to be orally administered, which provides significant benefits such as: lower active principle concentrations comprised in the formulation, smaller administered doses, therapeutic effect enhancement, pharmacological action speed, disease course modification, risk reduction for severe complications and risk reduction of manifesting secondary effects.

### DETAILED DESCRIPTION OF INVENTION

As we had previously mentioned, OA pharmacological treatment is divided into two groups: a) symptom modifying drugs, targeted for pain control and b) structure modifying drugs targeted to preserve joint cartilage and to slow down disease progress.
a) Symptom modifying drugs. *Fast action modifying drugs* are included within this group such as analgesics and non-steroidal antiinflammatories (NSAIDs), and *slow action modifying drugs,* named SYDADOA (Symtomatic Slow Action Drugs for Osteoarthritis).

### -Fast action modifying drugs.

*Analgesics.* When drugs are necessary for pain control, the first recommended is Paracetamol in a dose from 2 to 4 gr/day. This has demonstrated to be an efficient drug by controlling pain in 40% of patients, further being a safe drug.

Weak opiaceous analgesics (Tramadol and Codein) are efficient and safe drugs in arthrosic patient, provided manifested secondary effects are closely monitored especially in elderly people.

*Non-steroidal anti-inflammatories (NSAIDs).* Starting with an anti-inflammatory treatment is advised in patients who do not respond to non-pharmacological treatment neither to analgesics (paracetamol or weak opioids). NSAID treatment is prescribed from the beginning when data are available about joint inflammation, mainly synovial effusion. These shall be combined with gastroprotection by using a proton pump inhibitor because of a high risk of gastropathy associated with NSAIDs. Treatment with COX-2 inhibitors has the benefit of manifesting a lower gastropathy risk, but increases cardiovascular risk, therefore they shall be cautiously used in elderly people at low doses.

Application of creams or gels comprising NSAIDs (or capsaicin) is also an option shown to be efficient, specifically in knee arthrosis. They are recommended as supplementary treatment.

### -Slow action symptom modifying drugs.

This group is characterized because its effect starts after 2 to 3 weeks on treatment and is extended from 2 to 6 months after administration is ceased (remaining effect).

Hialuronic acid, Chondroitin sulphate, Diacerein and Glucosamine Sulphate are part of this therapeutic group. All of them have studies demonstrating their efficacy for arthrosis pain control, mainly in knee arthrosis.

Chondroitin is an antiarthritic with specific therapeutic activity. It is constituted by repeated molecule chains called glycosaminoglycans (GAG), mainly comprising a combination of several sulfated and non-sulfated glucuronic acid and n-acetyl-galactosamine residues which are needed for proteoglycan formation (which provide cartilage elasticity), being large macromolecules comprising a proteinaceous core where multiple glycosaminoglycan and oligosaccharide chains are adhered to, having a quite important structural function, as they retain water and several useful nutrients forming a healthy joint matrix, in addition to allowing the passage of other molecules therethrough, this being a very important property since cartilage is not supplied with blood. Its main function is performed as antiarthritic agent in patients having rheumatic diseases.

Chondroitin extraction and purification was firstly carried out around the 60's, this being a substance which is naturally present in the body, as the main cartilage constituent. Currently chondroitin is obtained and manufactured from natural sources such as: shark and/or cattle cartilage as well as from bovine trachea cartilage, which may be also obtained through synthetic processes. It is used to improve symptoms and to prevent progressive damage during degenerative process manifested by patients who suffer osteoarthritis.

Research experts declared along many years that orally administered chondroitin did not cause the desired effect, since molecules comprising them were too large being doubtful that they may be absorbed through digestive tract. However, researchers in 1995 ruled out above assumption based on evidence that up to 15% from orally administered chondroitin is absorbed intact through digestive tract.

Chondroitin is widely used in Europe for osteoarthritis treatment, since it protects joints from damage caused by progression thereof in addition to treating symptoms manifested by this disease.

Chondroitin is prevalent in tissue matrix such as: skin, bone, ligaments, tendons and blood vessels. Its properties are provided by collagen fiber orientation and glycosaminoglycan content.

Joint cartilage is a specialized tissue with a large extracellular expanded matrix which is responsible of 98% of its volumen, being only 2% at the expense of cells. There is initially an increase in proteoglican synthesis in OA, then impairment and exposure from collagen mesh to mechanical disruption.

Chondroitin sulphate is a proteoglican with a number of biological effects demonstrated under in vitro and in vivo studies. It is orally administered at doses of 800 mg/day. It has been demonstrated efficient in treatment of knee, hip and hand arthrosis. Its tolerance is good. Chondroitin sulphate is part of a large proteinaceous molecule (proteoglican) which provides elasticity to cartilage.

Once absorbed, chondroitin sulphate is incorporated in glycosaminoglycan rich-tissues, such as joint cartilage. It has been noticed that chondroitin sulphate action commencement is slower than other OA treatments, but its effects are prolonged longer after therapy withdrawal. Chondroitin sulphate clearance mainly occurs through renal route.

**Interleukin-1 selective inhibiting agents** act by blocking interleukin-1 activity produced by monocytes and macrophages as a immune system early response, stimulating T cell proliferation and protein synthesis.

Body's immune system which normally provides protection against harmful external influences may react mistakenly. Sometimes, immune system does not acknowledge body tissues as own and destroys those causing diseases. Among these "autoimmune diseases", rheumatoid arthritis is found.

Cytokines are messenger molecules which help cells to communicate therebetween. In rheumatoid arthritis, a defective immune system produces cytokines in excess which favor inflammation. One of the key cytokines in rheumatoid arthritis appearance process is a cytokine called interleukin-1 (IL-1). Interleukin-1 is released by emitter cells and is bound to specific receptors. This binding produces a number of reactions. Immune system induces IL-1 formation in synovial membrane where interleukin-1 causes inflammation and cartilage and bone destruction.

Interleukin-1 is a glycoproteinaceous nature molecule found in two biological forms, IL-1 alpha (IL-1α) and IL-1 beta (IL-1β). Both interleukins are bound to the same receptor in spite of not sharing high homology with aminoacid sequence (22%). There is another molecule similar to IL-1 beta bound to the same receptor but unlike the first ones, this does not manifest any biological activity. This feature has led to call it IL-1 (I-1Ra) receptor antagonist.

The three molecules constitute the IL-1 family. Genes that encode each of the members of this family are located in chromosome 2 long arm, site where genes are also located for two receptor types fixed to IL-1.

Immune system produces opposing molecules called antagonists, which are bound to the receptor preventing interleukin-1 attachment. In a healthy joint there is a sufficient amount of antagonists which control inflammatory process.

There is an imbalance between interleukin-1 and its antagonist in rheumatoid arthritis since there is a very high amount of interleukin-1 in the joint and very little amount of antagonist. Consequently, interleukin-1 disproportionally occupies many receptor sites thus maintaining joint inflammatory and destructive process.

Diacerein is a purified compound with antraquinonic structure showing and in vitro and in vivo inhibiting activity over interleukin-1 production and activity and metalloprotease secretion without altering prostaglandin synthesis, exerting a remodeling mechanism in joint cartilage.

Diacerein has demonstrated to be effective in osteoarthrosis (OA) treatment, a degenerative joint process characterized by progressive cartilage destruction and erosion. The use of Diacerein in animal models with OA as well as polyarthritis models (NZB/KN male mice), revealed that this consistently moderates cartilage degradation, preventing or reducing micro and macroscopic joint tissue injuries. Diacerein oral administration to patients with hip OA was associated with a symptomatic improvement and a cartilage structure modifying structure, together with a good safety profile.

Interleukin-1 is a factor intervening in macrophage-neutrophil response to inflammation and performs an important role in joint cartilage degradation. This factor together with TNF (Tumor Necrosis Factor) and other colony stimulating factors produce a retroaction mechanism commencing with tissue inflammation, followed by leukocyte formation.

A number of studies have demonstrated that Diacerein: inhibits interleukin-1 production and decreases collagenolytic activity.

Antiarthrosic diacerein properties are due to their capability of inhibiting pro-inflammatory and pro-catabolic cytokines, such as interleukin-1 which performs an important role in joint cartilage degradation, as well as in enzyme production and release inhibition which degrade cartilage (collagenase and stromelisin).

After being orally administered, Diacerein is hydrolyzed before penetrating into systemic circulation and suffering a first liver stage metabolism, being fully deacetylated and becoming into rein and its conjugates. Diacerein simultaneous ingestion with food causes a delay in its absorption by extending Tmax, and simultaneously increasing its bioavailability. Rein is bound to plasma proteins in 99%. Its excretion is through renal route in rein form and its conjugates (glucuronides and sulphates). Diacerin clearance half-life in plasma is from 5 to 7 hours.

Diacerein has a slow action start which is not significant until 4 weeks after treatment. The effect is maintained at least during 2 months after having cancelled the treatment.

There are currently a number of pharmaceutical products in marketplace directed to osteoarthrosis (OA) treatment, wherein several active principles are found which are independently formulated; however it is very important in this type of pathologies to reduce patient symptomatology including pain, inflammation, rigidity, as well as achieving a modification in disease course; therefore, the activity of a number of drugs acting through several routes is indispensable to reduce symptomatology, modifying the joint I cartilage structural disease course, reducing the risk of severe complications being present and decreasing the risk that secondary effects are manifested.

The pharmaceutical composition subject of present invention comprises a synergistic combination of an antiarthritic agent: Chondroitin sulphate and an interleukin-1 inhibiting agent: Diacerein, in addition to pharmaceutically acceptable excipients, which are formulated in a single dosage unit to be orally administered, for use in the control and treatment of osteoarthrosis.

Said pharmaceutical composition has been developed taking into account that both active principles have a great efficacy and capacity for rheumatoid arthritis treatment, further achieving a reduction in inflammatory process, pain, and a modification in disease course at joint cartilage structural level.

Antiarthritic agent used in the pharmaceutical composition subject of the present invention : Chondroitin sulphate, is present in the formulation in a concentration range from 100.0 mg to 1.2 g per dose unit. I

Interleukin-1 inhibiting agent used in the pharmaceutical composition subject of present invention: Diacerein, is present in the formulation in a concentration range from 25.0 mg to 100.0 mg per dose unit.

In order to assess the efficacy of the pharmaceutical composition subject of present invention, as well as the synergistic effect resulting from a combination of active principles: Diacerein and Chondroitin sulphate in a single dosage unit, a clinical comparative study was carried out wherein above mentioned active principles were separately administered, as well as a combination thereof.

### CLINICAL EFFICACY STUDY OF A COMBINATION DIACEREIN / CHONDROITIN VS. DIACEREIN AND CHONDROITIN INDEPENDENTLY ADMINISTERED.

A comparative clinical study in patients with hip OA was carried out with a 6 month term for follow-up. The study compared the efficacy of single Diacerein administration, single chondroitin and a combination of Diacerein/Chondroitin. The study included 120 patients which were divided in 3 treatment groups:
Group 1: received Diacerein 50 mg administration twice a day.
Group 2: received Chondroitin 600 mg administration twice a day.
Group 3 : received Diacerein 50 mg /Chondroitin 600 mg administration twice a day.

Patients fulfilled with the inclusion criteria from the American Rheumatology Academy. Inclusion criteria were:
- Symptomatic disease presence, defined as daily hip pain at least during the last two months.
- A functional pain Lequesne index at least of 3 points.
- Radiographic criteria were a joint space between 1 and 3 mm (without exceeding 3 mm) or less than 0.5 mm qf contralateral hip.

Joint hip space was measured through a prior pelvic radiography reading center, using a graded 0.1 mm magnification crystal. Start and end of treatment was radiographically assessed comparing the affected vs contralateral hip.

Laboratory analyses were carried out at the beginning of the study and on 6 months of treatment, assessing liver and kidney performance.

### RESULTS

Baseline characteristics were compared among the 3 groups of treatment.

Group 1: Forty patients.

Group 2: Forty patients.

Group 3: Forty patients.

After two months of treatment, 6 patients from Group 1 (Diacerein) cancelled the treatment (because of inefficacy).

Eight patients from Group 2 (Chondroitin) cancelled the treatment (because of inefficacy).

No patient from Group 3 cancelled the treatment during the 6 follow-up months.

**Table 1. Baseline Characteristics**

| Characteristics | Groups of Treatment | | |
|---|---|---|---|
| | Group 1 (n=34) | Group 2 (n=32) | Group 3 (n=40) |
| Average age ± SD (years) | 62.1 ± 7.0 | 63.0 ± 6.7 | 61.2 ± 6.0 |
| Male sex (%) | 34 | 32 | 40 |
| Body Mass Index (IMC) average ± SD (Kg/m²) | 25.4 ± 2.3 | 26.1 ± 3.0 | 26.0 ± 3.5 |
| Average disease period ± SD | 4.8 ± 4.7 | 4.6 ± 4.3 | 5.1 ± 5.6 |
| OA location in patients (%) | | | |
| Superolateral | | | |
| Superomedial | 48 | 54 | 56 |
| Concentric | 24 | 26 | 25 |
| No joint space | 08 | 10 | 12 |
| | 20 | 10 | 7 |
| Average symptom severity (EVA) ± SD (mm) | 46 ± 18 | 45 ± 20 | 43 ± 22 |
| | | | |

Patient percentage with radiographic progression was defined as a joint space loss at least of 0.5 mm, which was significantly lower in group 3 patients compared with Groups 1 and 2 patients.

Data from first laboratory analysis: 51.6% vs. 61.3% vs. 60.2% [P= 0.035]

Data upon 6 month treatment completion: 46.7% vs. 61.2 vs. 43.6 [P= 0.006].

As to those objectives which were met at 6 months of treatment regarding the joint space:
Group 1: Average ± SD 0.17 ± 0.24 mm/6 months.
Group 2: Average ± SD 0.22 ± 0.22 mm/6 months.
Group 3: Average ± SD 0.14 ± 0.20 mm/6 months.

### CONCLUSIONS

The 6 month study showed a modifying effect on hip OA structure in all treatment groups; however, the treatment with a combination of Diacerein/Chondroitin was better than the other two groups.

All drugs were well tolerated; however, treatment cancellation in some patients from Groups 1 and 2 was due to a slowness of patient symptomatology effects.

## Claims

1. Pharmaceutical composition **characterized in that** the composition comprises a synergistic combination of an antiarthritic agent, namely Chondroitin sulphate, and an interleukin-1 inhibiting agent, namely Diacerein, in addition to pharmaceutically acceptable excipients; wherein Chondroitin sulphate is present in the formulation in a concentration range from 100.0 mg to 1.2 g and Diacerein from 25.0 mg to 100.0 mg, which are formulated in a single dosage unit to be orally administered, for use in the control and treatment of Osteoarthrosis.

2. Pharmaceutical composition for use according to claim 1, **characterized in that** Chondroitin sulphate is present in the formulation in a concentration of 600.0 mg per dose unit.

3. Pharmaceutical composition for use according to claims 1 and 2, **characterized in that** Diacerein is present in the formulation in a concentration of 50.0 mg per dose unit.

4. Pharmaceutical composition for use according to claims 1 to 3, **characterized in that** the composition is formulated in a single dosage unit to be orally administered in capsule, tablet or sachet form.

## Patentansprüche

1. Pharmazeutische Zusammensetzung, **dadurch gekennzeichnet, dass** die Zusammensetzung eine synergistische Kombination aus einem Antarthritikum, nämlich Chondroitinsulfat, und einem Interleukin-1 hemmendes Mittel, nämlich Diacerein, zusätzlich zu pharmazeutisch verträglichen Hilfsstoffen, umfasst; wobei Chondroitinsulfat in der Formulierung in einem Konzentrationsbereich von 100,0 mg bis 1,2 g vorhanden ist und Diacerein von 25,0 mg bis 100,0 mg, welche in einer einzigen Dosierungseinheit formuliert sind, um oral verabreicht zu werden, für den Einsatz in der Kontrolle und Behandlung von Osteoarthrose.

2. Pharmazeutische Zusammensetzung für den Einsatz nach Anspruch 1, **dadurch gekennzeichnet, dass** Chondroitinsulfat in der Formulierung in einer Konzentration von 600,0 mg pro Dosiseinheit vorhanden ist.

3. Pharmazeutische Zusammensetzung für den Einsatz nach den Ansprüchen 1 und 2, **dadurch gekennzeichnet, dass** Diacerein in der Formulierung in einer Konzentration von 50,0 mg pro Dosiseinheit vorhanden ist.

4. Pharmazeutische Zusammensetzung für den Einsatz nach Ansprüchen 1 bis 3, **dadurch gekennzeichnet, dass** die Zusammensetzung in einer einzigen Dosierungseinheit formuliert ist, um oral in Kapsel-, Tabletten- oder Sachetform verabreicht zu werden.

## Revendications

1. Composition pharmaceutique **caractérisée en ce que** la composition comprend une combinaison synergistique d'un agent antiarthritique, à savoir le sulfate de chondroïtine, et un agent inhibiteur d'interleukin-1, à savoir la diacérein, en plus des excipients pharmaceutiquement acceptables ; dans laquelle le sulfate de chondroïtine est présent dans la formule dans une plage de concentration comprise entre 100,0 mg et 1,2 g et la diacérein comprise entre 25,0 mg et 100,0 mg, qui sont formulés dans une seule unité de dosage destinée à être oralement administrée, pour une utilisation dans le contrôle et le traitement de l'ostéoarthrose.

2. Composition pharmaceutique pour une utilisation selon la revendication 1, **caractérisée en ce que** le sulfate de chondroïtine est présent dans la formule dans une concentration de 600,0 mg par unité de dosage.

3. Composition pharmaceutique pour une utilisation selon les revendications 1 et 2, **caractérisée en ce que** la diacérein est présente dans la formule dans une concentration de 50,0 mg par unité de dosage.

4. Composition pharmaceutique pour une utilisation selon les revendications 1 à 3, **caractérisée en ce que** la composition est formulée dans une seule unité de dosage destinée à être oralement administrée sous forme de gélule, comprimé ou sachet.
